# EUROPEAN PATENT APPLICATION

(11) **EP 4 353 238 A1**
(43) Date of publication of application: **17.04.2024**
(21) Application number: 22820463.2
(22) Date of filing: 31.05.2022
(51) Int. Cl.: A61K 31/7008, A61P 43/00, A61P 3/04, A23L 33/125, A23K 20/163

(54) **COMPOSITION FOR IMPROVING INTESTINAL MICROBIAL POPULATION COMPRISING GALACTOSE**

(30) Priority: 11.06.2021 KR 20210076344
(71) Applicant: Quorum Bio Co., Ltd, Seoul 03080 (KR)
(72) Inventor: SIM, Jae Hyun, Seoul 07988 (KR); RYU, Eunju, Seoul 07703 (KR); KIM, Yunji, Seoul 01701 (KR); KIM, Hye Eun, Seoul 05033 (KR); SIM, Jun, Incheon 21596 (KR)
(74) Representative: Delbarba, Andrea
(86) International application number: PCT/KR2022/007721
(87) International publication number: WO 2022/260335

(57) **Abstract**

Provided are: a pharmaceutical composition, food composition, or feed composition for improving the intestinal microbial population comprising galactose, and for reducing body weight; and use thereof for preventing and/or treating and/or ameliorating obesity.

## Description

### [TECHNICAL FIELD]

### CROSS-REFERENCE TO RELATED APPLICATION(S)

This application claims priority benefit of Korean Patent Application No. 10-2021-0076344 filed on June 11, 2021 with the Korean Intellectual Property Office, the contents of which are incorporated herein by reference in its entirety.

Provided is a pharmaceutical composition, food composition or feed composition for improving the intestinal microbial population and for reducing body weight, each comprising galactose; and a use thereof for preventing and/or treating and/or ameliorating obesity.

### [BACKGROUND ART]

Galactose is a kind of monosaccharide, which is mainly used as a sweetener, and is not well known for its application except a sweetener. In recent years, it has been discovered that by suppressing quorum sensing of oral bacteria, biofilm formation can be suppressed, and oral diseases such as dental caries and periodontal disease can be effectively suppressed.

However, until now, research on galactose has been limited only to the oral cavity, and little research has been conducted on how it affects intestinal bacteria. Not only the intestinal bacteria has a more complex composition than the oral bacteria, but also it is difficult to collect specimens and observe phenotypes, which poses many limitations to research.

### [DETAILED DESCRIPTION OF THE INVENTION]

### [Technical Problem]

Provided herein is a use of a composition comprising galactose for improving the intestinal microbial population and/or for reducing body weight (weight loss), and/or for preventing, ameliorating and/or treating obesity. One embodiment of the invention provides a use of galactose for improving the intestinal microbial population and/or for reducing body weight (weight loss), and/or preventing, ameliorating and/or treating obesity.

Specifically, one embodiment of the invention provides a composition for improving the intestinal microbial population, comprising galactose.

Another embodiment of the invention provides a pharmaceutical composition for reducing body weight, comprising galactose as an active ingredient.

A further embodiment of the invention provides a pharmaceutical composition for preventing and/or treating obesity, comprising galactose as an active ingredient.

Still another embodiment of the invention provides a food composition for reducing body weight, comprising galactose as an active ingredient.

Yet another embodiment of the invention provides a feed composition for reducing body weight, comprising galactose as an active ingredient.

A yet further embodiment of the invention provides a feed composition for preventing and/or ameliorating obesity, comprising galactose as an active ingredient.

Another embodiment of the invention provides a method for improving the intestinal microbial population, comprising administering galactose to an individual (subject) in need thereof.

Even another embodiment of the invention provides a method for reducing body weight, comprising administering a pharmaceutically effective amount of galactose to an individual (subject) in need thereof.

Still yet another embodiment of the invention provides a method for preventing, ameliorating and/or treating obesity, comprising administering a pharmaceutically effective amount of galactose to an individual (subject) in need thereof.

Even yet another embodiment of the invention provides a use of galactose in the preparation of a pharmaceutical composition for reducing body weight, a pharmaceutical composition for preventing and/or treating obesity, a food composition for reducing body weight, a food composition for preventing and/or ameliorating obesity, a feed composition for reducing body weight, or a feed composition for preventing and/or ameliorating obesity.

### [Technical Solution]

In the present invention, metagenomic analysis has been performed by orally administering galactose to a laboratory animal (mouse) induced with obesity by a high-fat diet, and then collecting feces, and as a result, it has been found that changes appear in the proportion of intestinal microorganisms associated with obesity. Thereby, provided are uses of galactose for improving the intestinal microbial population and/or for reducing body weight (weight loss) and/or for preventing, ameliorating and/or treating obesity.

As used herein, 'galactose' is a monosaccharide containing 6 carbon atoms, which is an aldose having an aldehyde group, and its chemical formula is C₆H₁₂O₆. Galactose and glucose are epimers that are different in stereochemical properties at the fourth carbon (C4). Galactose used herein may be D-galactose, and may be e in the form of a monomer that is not oligomerized and polymerized.

One aspect of the invention provides a composition for improving the intestinal microbial population, comprising galactose as an active ingredient.

The phrase "improving the intestinal microbial population" may mean increasing beneficial intestinal bacteria as compared with a control group (a group before administration of the composition or without administration of the composition). The increase in beneficial intestinal bacteria may mean an increase in the population of the beneficial intestinal bacteria and/or an increase in the proportion thereof (beneficial intestinal bacterial population/total intestinal bacterial population).

Therefore, the composition for improving the intestinal microbial population may be characterized by increasing the proportion (or population) of beneficial intestinal bacteria as compared with a control group (a group before administration of the composition or without administration of the composition).

The intestinal microorganisms may mean all microorganisms existing in the intestines, and may include one or more kinds of microorganisms selected from the group consisting of *Actinobacteria* (*Actinomyces*), *Bacteroidetes, Candidatus melainabacteria, Cyanobacteria, Deferribacteres, Firmicutes, Proteobacteria, Tenericutes, Verrucomicrobia, Akkermansia, Lactobacillus, Alistipes,* and the like.

In one embodiment, the intestinal microorganism may be one or more kinds of microorganisms selected from the group consisting of *Akkermansia, Alistipes,* and *Lactobacillus.*

The beneficial intestinal bacteria are bacteria that have the effect of reducing body weight, and/or preventing, ameliorating and/or treating obesity among the intestinal microorganisms. The beneficial intestinal bacteria may include one or more (or two or more, three or more, or all) bacteria selected from the group consisting of *Akkermansia* bacteria, *Lactobacillus* bacteria, and *Alistipes* bacteria. In one embodiment, the beneficial intestinal bacteria may be bacteria of the genus *Akkermansia.*

The population of the bacteria (total intestinal microorganisms or beneficial intestinal bacteria) can be confirmed by methods such as metagenomic analysis and metagenomic sequencing after collecting samples (e.g., feces) from a subject (individual) administrated with the composition, but is not limited thereto.

Another aspect provides a composition (e.g., a pharmaceutical composition, food composition, or feed composition, etc.) for reducing body weight, comprising galactose as an active ingredient.

Another aspect provides a composition (e.g., a pharmaceutical composition, food composition, or feed composition, etc.) for preventing, ameliorating and/or treating obesity, comprising galactose as an active ingredient.

The expression "reducing body weight" or "preventing, ameliorating and/or treating obesity" may mean increasing in the proportion (or population) of beneficial intestinal bacteria as compared with a group before administration of the composition or without administration of the composition.

Therefore, the composition is characterized by increasing the proportion (or population) of beneficial intestinal bacteria as compared with a control group (a normal weight subject, a group before administration of the composition or without administration of the composition), thereby capable of achieving the effect of reducing body weight and/or preventing, ameliorating and/or treating obesity.

The beneficial intestinal bacteria are bacteria that have the effect of reducing body weight and/or preventing, ameliorating and/or treating obesity, and the beneficial intestinal bacteria may include one or more (two or more, three or more, or all) bacteria selected from the group consisting of *Akkermansia* bacteria, *Lactobacillus* bacteria and *Alistipes* bacteria, but are not limited thereto.

Therefore, In one embodiment, there is provided a composition for improving the intestinal microbial population, a pharmaceutical composition for reducing body weight, a pharmaceutical composition for preventing and/or treating obesity, a food composition for reducing body weight, a food composition for preventing and/or ameliorating obesity, a feed composition for reducing body weight, or a feed composition for preventing and/or ameliorating obesity, characterized in that the composition comprises galactose as an active ingredient, and increases the proportion (or increasing the ratio) of one or more (e.g., one, two, or all three) beneficial intestinal bacteria selected from the group consisting of *Akkermansia* bacteria, *Lactobacillus* bacteria, and *Alistipes* bacteria.

In another embodiment, there is provided a composition (a composition for improving the intestinal microbial population, a pharmaceutical composition, food composition, and/or a feed composition for reducing body weight, and/or for preventing, ameliorating and/or treating obesity), which comprises galactose as an active ingredient and increases the proportion of bacteria of the genus *Akkermansia.*

The composition may be characterized by increasing the proportion (or population) of bacteria of genus *Akkermansia,* and further increasing the proportion of one or more (e.g., one or both) selected from the group consisting of *Lactobacillus* bacteria and *Alistipes* bacteria.

The composition may be characterized in that it is administered to a subject in which the proportion of one or more (e.g., one, two, or all three) beneficial intestinal bacteria selected from the group consisting of *Acamansia* bacteria, *Lactobacillus* bacteria, and *Alistipes* bacteria is reduced as compared with a normal weight subject.

In one embodiment, the composition may be characterized in that it is administered to a subject in which the proportion of bacteria of the genus *Akkermansia* is reduced as compared with a normal weight subject.

The composition may be characterized in that it is administered to a subject in which the proportion of bacteria of the genus Akkermansia is reduced as compared with a normal weight subject, and further, the proportion of one or more (e.g. , one, or both) selected from the group consisting of *Lactobacillus* bacteria and *Alistipes* bacteria (e.g. , one, or both) is reduced.

Specifically, the composition is characterized by increasing the proportion of bacteria of the genus *Akkermansia* among beneficial intestinal bacteria, and the composition may be for administering to a subject in which the proportion of bacteria of the genus *Akkermansia* among beneficial intestinal bacteria is reduced as compared with a normal weight subject.

The composition may be characterized by further increasing the proportion of *Alistipes* bacteria among beneficial intestinal bacteria.

Increasing the amount, population, and/or proportion of the beneficial intestinal bacteria has the effect of reducing body weight and preventing, ameliorating and/or treating obesity.

As used herein, the term "treatment" is used to encompass alleviation or amelioration of pathological symptoms, reduction of the disease site, delay or alleviation of disease progression, amelioration, alleviation or stabilization of disease state or symptoms, partial or complete recovery, prolonged survival, other beneficial treatment results, and the like. The term "prevention" is used to encompass all mechanisms and/or effects that act on a subject not having a specific disease to prevent the onset of a specific disease or delay the onset of the disease.

The pharmaceutical composition used herein may be administered to mammals comprising humans, dogs, cats, horses, cattle, pigs, goats, rabbits, mice, rats, etc., or cells or tissues separated therefrom or cultures thereof via a variety of routes. The administration may be performed via any route commonly used, for example, oral administration or parenteral administration such as intravenous administration, intramuscular administration, subcutaneous administration, intraperitoneal administration, and topical administration. Particularly, the administration may be performed via an oral administration. The pharmaceutical composition may be formulated into oral preparations such as a powder, a granule, a tablet, a capsule, a suspension, an emulsion, a syrup, an aerosol, etc. according to the common methods.

The pharmaceutical composition may further comprise a pharmaceutically suitable and physiologically acceptable additive such as a carrier, an excipient, and/or a diluent, in addition to galactose as the active ingredient. Examples of the carrier, excipient, or diluent may comprise one or more selected from the group consisting of lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oils. Such preparations may be formulated using one or more diluents or excipients selected from the group consisting of a filler, an extender, a binder, a wetting agent, a disintegrating agent, and a surfactant ordinarily employed. Examples of the solid preparation for oral administration comprise one or more selected from the group consisting of a tablet, a pill, a powder, a granule, a capsule, a syrup, a powder, a suspension, etc., and the solid preparation may be prepared by mixing galactose with one or more excipients, for example, selected from the group consisting of starch, calcium carbonate, sucrose, lactose, and gelatin. Further, in addition to the simple excipients, lubricants such as magnesium stearate and talc may also be used. Examples of a liquid preparation for oral administration may comprise one or more selected from the group consisting of a suspension, a liquid for internal use, an emulsion, and a syrup. Various excipients, for example, one or more selected from the group consisting of a wetting agent, a sweetener, a flavor, and a preservative may be comprised, in addition to commonly used simple diluents such as water and liquid paraffin.

A pharmaceutical composition or galactose as an active ingredient can be administered in a pharmaceutically effective amount. An administration dose of the pharmaceutical composition or galactose as an active ingredient may vary depending on factors such as a formulation method, administration mode, a patient's age, body weight, gender, health state, and diet, administration time, administration frequency, administration routes, excretion rates, and responsiveness. The administration dose may vary depending on a patient's age, body weight, gender, administration mode, health condition, and disease severity, and the pharmaceutical composition may be administered in a single dose or divided into several doses per day at a predetermined time interval according to the instructions of a physician or a pharmacist. For example, the once or daily dose of the pharmaceutical composition may be in the range of 0.001 to 10000 mg/kg, specifically, 0.01 to 10000 mg/kg, 0.01 to 5000 mg/kg, 0.01 to 3000 mg/kg, 0.01 to 2500 mg/kg, 0.01 to 2000 mg/kg, 0.01 to 1500 mg/kg, 0.01 to 1000 mg/kg, 0.01 to 500 mg/kg, 0.01 to 300 mg/kg, 0.01 to 200 mg/kg, 0.1 to 10000 mg/kg, 0.1 to 5000 mg/kg, 0.1 to 3000 mg/kg, 0.1 to 2500 mg/kg, 0.1 to 2000 mg/kg, 0.1 to 1500 mg/kg, 0.1 to 1000 mg/kg, 0.1 to 500 mg/kg, 0.1 to 300 mg/kg, 0.1 to 200 mg/kg, 0.1 to 100 mg/kg, 0.1 to 50 mg/kg, 0.1 to 30 mg/kg, 0.1 to 20 mg/kg, 0.1 to 10 mg/kg, 1 to 10000 mg/kg, 1 to 5000 mg/kg, 1 to 3000 mg/kg, 1 to 2500 mg/kg, 1 to 2000 mg/kg, 1 to 1500 mg/kg, 1 to 1000 mg/kg, 1 to 500 mg/kg, 1 to 300 mg/kg, 1 to 200 mg/kg, 1 to 100 mg/kg, 1 to 50 mg/kg, 1 to 30 mg/kg, 1 to 20 mg/kg, 0.1 to 10 mg/kg, 10 to 10000 mg/kg, 10 to 5000 mg/kg, 10 to 3000 mg/kg, 10 to 2500 mg/kg, 10 to 2000 mg/kg, 10 to 1500 mg/kg, 10 to 1000 mg/kg, 10 to 500 mg/kg, 10 to 300 mg/kg, 10 to 200 mg/kg, 10 100 mg/kg, 10 to 50 mg/kg, 10 to 30 mg/kg, 10 to 20 mg/kg, 30 to 10000 mg/kg, 30 to 5000 mg/kg, 30 to 3000 mg/kg, 30 to 2500 mg/kg, 30 to 2000 mg/kg, 30 to 1500 mg/kg, 30 to 1000 mg/kg, 30 to 500 mg/kg, 30 to 300 mg/kg, 30 to 200 mg/kg, 30 to 100 mg/kg, 30 to 50 mg/kg, 50 to 10000 mg/kg, 50 to 5000 mg/kg, 50 to 3000 mg/kg, 50 to 2500 mg/kg, 50 to 2000 mg/kg, 50 to 1500 mg/kg, 50 to 1000 mg/kg, 50 to 500 mg/kg, 50 to 300 mg/kg, 50 to 200 mg/kg, or 50 to 100 mg/kg, based on a weight of the active ingredient (galactose), but is not limited thereto. The once or daily dose may be prepared as a single formulation in a unit dosage form, or prepared in an appropriate divided dosage form, or prepared in multi-unit dosage forms. The above-mentioned doses are exemplary of the average case, and the doses may be higher or lower depending on individual differences.

The above food composition is a food produced by using nutrients that are easily deficient in daily meals and raw materials that have functions useful for the human body (hereinafter referred to as "functional raw materials"), and refers to any food that helps maintain health or prevent and/or ameliorate (alleviate) certain diseases or symptoms, and the final product form is not particularly limited. For example, the food composition may be selected from the group consisting of various foods, health functional foods, beverage compositions, food additives, etc., but is not limited thereto.

The content of galactose as an active ingredient contained in the food composition is not particularly limited, but can be used appropriately depending on the form of the food, desired use, and the like. For example, the content of galactose may be 0.001 to 99 % by weight, 0.01 to 99 % by weight, 0.01 to 95 % by weight, 0.01 to 90 % by weight, 0.01 to 80 % by weight, 0.01 to 50 % by weight, 0.1 to 99 % by weight, 0.1 to 95 % by weight, 0.1 to 90 % by weight, 0.1 to 80 % by weight, 0.1 to 50 % by weight, 1 to 99 % by weight, 1 to 95 % by weight, 1 to 90 % by weight, 1 to 80 % by weight, 1 to 50 % by weight, 10 to 99 % by weight, 10 to 95 % by weight, 10 to 90 % by weight, 10 to 80 % by weight, 10 to 50 % by weight, 25 to 99 % by weight, 25 to 95 % by weight, 25 to 90 % by weight, 25 to 80 % by weight, 25 to 50 % by weight, 40 to 99 % by weight, 40 to 95 % by weight, 40 to 90 % by weight, 40 to 80 % by weight, 40 to 50 % by weight, 50 to 99 % by weight, 50 to 95 % by weight, 50 to 90 % by weight, 50 to 80 % by weight, 60 to 99 % by weight, 60 to 95 % by weight, 60 to 90 % by weight, or 60 to 80 % by weight, based on the total weight of the food.

The food composition may further comprise one or more kinds selected from the group consisting of various nutrients, vitamins, minerals (electrolytes), flavoring agents such as synthetic flavors or natural flavors, coloring agents, improving agents (cheese, chocolate, etc.), pectic acid or salts thereof, alginic acid or salts thereof, organic acids, protective colloidal thickening agents, pH controlling agents, stabilizing agents, preservatives, glycerin, alcohol, carbonating agent used in carbonated drinks, etc. The proportion of such additives is generally selected from the range of 0.001 to about 20 parts by weight per 100 parts by weight of the total food composition, but is not limited thereto.

In one embodiment, the composition (e.g., pharmaceutical composition, food composition, feed composition, etc.) may be characterized by administrating to a subject (e.g., individual, patient, etc.) having a reduced proportion (or population) of the beneficial intestinal bacteria as compared with a normal weight subject (e.g., individual, patient, etc.).

The proportion of beneficial intestinal bacteria means the number of intestinal beneficial bacteria compared to the number of total intestinal bacteria, and the reduction in the proportion (or population) of the beneficial intestinal bacteria means that the proportion (or population) of the beneficial intestinal bacteria is reduced as compared with the proportion (or population) of beneficial intestinal bacteria in a control group (non-obese; normal weight subject).

Yet another aspect of the invention provides a method for improving the intestinal microbial population, comprising administering galactose to an individual (subject) in need thereof.

A yet further aspect of the invention provides a method for reducing body weight or a method for preventing and/or treating obesity, comprising administering a pharmaceutically effective amount of galactose to an individual (subject) in need thereof.

### [Advantageous Effects]

In the present invention, it has been confirmed that galactose causes changes in the proportion of intestinal microorganisms associated with obesity, and based on such effects, a composition for improving the intestinal microbial population or for reducing body weight or for preventing, ameliorating and/or treating obesity, comprising galactose is provided.

### [BRIEF DESCRIPTION OF THE DRAWINGS]

FIG. 1 is a schematic diagram showing the preparation of laboratory animals and the animal testing process in one embodiment.
FIG. 2 is a graph showing that during galactose ingestion of mice induced with obesity by a high-fat diet, the proportion of beneficial intestinal bacteria increased compared to the control group not ingested with galactose.
FIG. 3 is a graph showing the weight gain during galactose ingestion of mice induced with obesity by high-fat diet, as compared with a control group not ingested with galactose.
FIG. 4 is a graph showing the blood glucose level upon galactose ingestion of mice induced with obesity by a high-fat diet, as compared with a control group not ingested with galactose.
FIG. 5 is a graph showing the Area Under Curve of the graph of FIG. 4.

### [DETAILED DESCRIPTION OF THE EMBODIMENTS]

Hereinafter, the present invention will be described in detail with reference to the following Examples. However, these Examples are for illustrative purposes only, and the invention is not intended to be limited by these Examples.

### Example 1. Preparation of feed and experimental animals

### 1.1. Preparation of Galactose

After lactose was separated into glucose and galactose using lactose hydrolase, galactose was separated and purified, and moisture was removed and powdered to prepare a galactose sample, which was used in the following test.

### 1.2. Preparation of Experimental Animal

In this specification, animal experiments were performed in an environment maintained at a humidity of 50 ± 5% and a temperature of 22 ± 2°C in accordance with the rules prescribed by the IACUC of Chonnam National University.

A 5-week-old male C57BU6J mouse (purchased from Damul Science Co.) bred as an experimental animal was prepared, acclimatized in a laboratory environment for one week while supplying sufficient food and water, and then used in the experiment. The straw litter used was non-allergenic, dust-free, nontoxic, absorbent, and pathogen-free. The cages were changed once a week to keep the animals clean, and water was supplied ad libitum in water bottles.

### 1.3. Preparation and Test Design of High-Fat-Diet Induced Obesity Animal Model

A 5-week-old male C57BL/6J mouse prepared in Example 1.2 were used after a 2-week acclimatization process, and then adjustment of body weight for each group. From the time the mouse reached 7 weeks of age, obesity was induced with a high fat diet (HFD).

The fat content of the high-fat-diet feed was set to about 60 wt.%, and the fat content of the general feed was set to about 10 wt.%.

The high-fat diet feed (D12492) was calculated as 5.24 kcal per 1g, and the general feed (D12450B) was calculated as 3.82 kcal per 1g.

The mice were fed ad libitum with a general feed or a high-fat diet feed for 16 weeks, and PBS and galactose were orally administered daily to each group. The input amount and the remaining amount of the feed were measured twice a week to calculate daily caloric intake, and the body weight was measured once a week at a designated time.

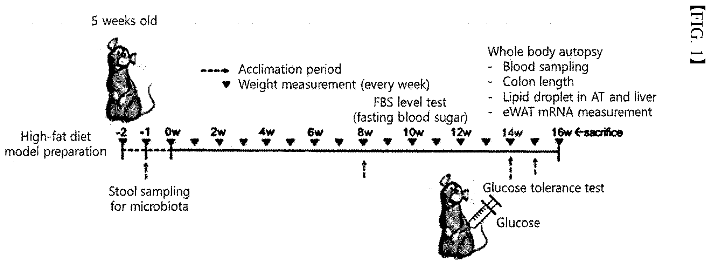

For microbiome analysis, feces were collected as shown in FIG. 1 and analysis was requested to Macrogen Inc. Feces were collected after selecting five mice whose body weight was closest to the average.

The ingredients of the high-fat diet feed and the general feed used above are shown in Table 1 below.

**[Table 1]**

| **Product#** | **D12450B** | | **D12492** | |
|---|---|---|---|---|
| | gm% | *kcal%* | gm% | *kcal%* |
| Protein | 19.2 | *20.0* | 26.2 | *20.0* |
| Carbohydrate | 67.3 | *70.0* | 26.3 | *20.1* |
| Fat | 4.3 | *10.0* | 34.9 | *59.9* |
| Total | | *100.0* | | *100.0* |
| kcal/gm | 3.85 | | 5.24 | |
| | | | | |

| **Ingredient** | **gm** | ***kcal*** | **gm** | ***kcal*** |
|---|---|---|---|---|
| Casein, 80 Mesh | 200 | *800* | 200 | *800* |
| L-Cystine | 3 | *12* | 3 | *12* |
| | | | | |
| Corn Starch | 315 | *1260* | 0 | 0 |
| Maltodextrin 10 | 35 | *140* | 125 | *500* |
| Sucrose | 350 | *1400* | 68.8 | *275.2* |
| | | | | |
| Cellulose, BW200 | 50 | *0* | 50 | *0* |
| | | | | |
| Soybean Oil | 25 | *225* | 25 | *225* |
| Lard | 20 | *180* | 245 | *2205* |
| | | | | |
| Mineral Mix, S10026 | 10 | *0* | 10 | *0* |
| Dicalcium Phosphate | 13 | *0* | 13 | *0* |
| Calcium Carbonate | 5.5 | *0* | 5.5 | *0* |
| Potassium Citrate, 1H2O | 16.5 | *0* | 16.5 | *0* |
| | | | | |
| Vitamin Mix, V10001 | 10 | *40* | 10 | *40* |
| Choline Bitartrate | 2 | *0* | 2 | *0* |
| | | | | |
| FD&C Yellow Dye #5 | 0.05 | *0* | 0 | *0* |
| FD&C Red Dye #40 | 0 | *0* | 0 | *0* |
| FD&C Blue Dye #1 | 0 | *0* | 0.05 | *0* |
| **Total** | **1055.05** | ***4057*** | **773.85** | ***4057*** |

Experimental animals were classified as shown in Table 2 below and administered with PBS or galactose:

**[Table 2]**

| Group | Diet | Drug administration (oral administration) | Administration dose | Populati on |
|---|---|---|---|---|
| 1 | Normal diet | PBS | - | 8 |
| 2 | Normal diet | Galactose | 200mg/kg | 8 |
| 3 | High fat diet | PBS | - | 8 |
| 4 | High fat diet | Galactose | 200mg/kg | 8 |
| 5 | High fat diet | Galactose | 100mg/kg | 8 |
| 6 | High fat diet | Galactose | 50mg/kg | 8 |
| Total | | | | 48 |

### Example 2. Galactose Microbiome Analysis in a High-Fat Diet Induced Obesity Model

The number of animals in each group was set to 8. After 8 weeks of the experiment, metagenome analysis was performed by collecting feces from the 3^{rd} group (HFD-PBS), the 5^{th} group (HFD-Gal 100mg/kg), and the 6^{th} group (HFD-Gal 50mg/kg). The results of the metagenomic analysis were obtained by requesting metagenome sequencing to Macrogen Co., Ltd.

As beneficial intestinal bacteria, bacteria belonging to the genus *Akkermansia, Lactobacillus,* and *Alistipes* are known as genera that can improve obesity.

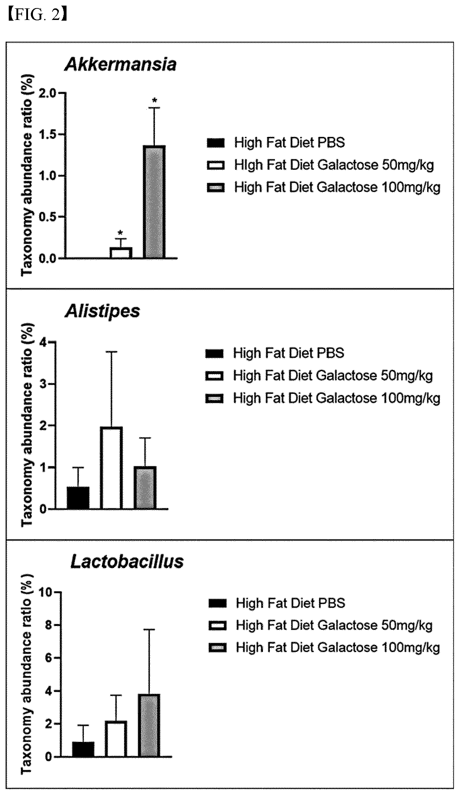

Among the intestinal microorganisms, an analysis of the composition of obesity-related intestinal microorganisms by the genus is shown in FIG. 2 and Table 3 below.

**[Table 3]**

| Group | *Akkermansia* | | *Lactobacillus* | | *Alistipes* | |
|---|---|---|---|---|---|---|
| | Average (%) | Standard deviation | Average (%) | Standard deviation | Average (%) | Standard deviation |
| 3 | 0 | 0 | 0.885 | 0.886524 | 0.54 | 0.397681 |
| 6 | 0.135 | 0.183644 | 2.17 | 1.36413 | 1.9675 | 1.564726 |
| 5 | 1.365 | 0.794056 | 3.8375 | 3.377554 | 1.0275 | 0.5880201 |

As shown in FIG. 2 and Table 3, the galactose-administered group increased in the proportion of *Akkermansia, Lactobacillus,* and *Alistipes* as compared with a control group.

For the experimental results, significance was tested using the TTEST method, and when the P value was less than 0.05, that value was determined to be significant ((*)<0.05, (**)<0.01, (***)<0.001).

### Example 3. Measurement of Body Weight

During the experimental period (16 weeks) designed in Example 1.3, the body weight of the experimental animals were measured at a predetermined time every week before administration of galactose or PBS, and body weight change (weight gain) and survival rate were measured (allowed to euthanize if the body weight decreases to less than 20% of the initial body weight). The weight gain was calculated by setting the body weight (initial body weight) of the experimental animal at 7 weeks of age as 100% and then calculating the body weight measured at each time point as a relative value to the initial body weight.

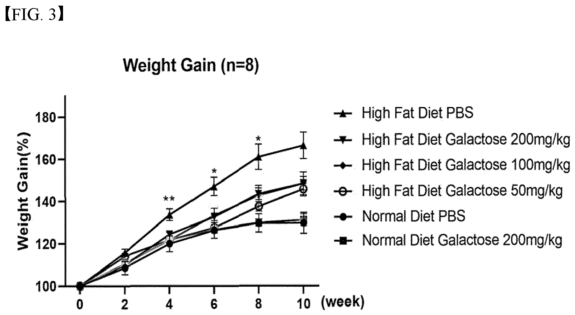

The average value of the weight gain (n=8) thus obtained is shown in FIG. 3. As shown in FIG. 3, it was confirmed that in a general diet group, no significant difference was observed between the weight gains of the PBS-administered group and the galactose-administered group, whereas in the high-fat diet feed group, the weight gain of the galactose-administered group was significantly reduced as compared with a control group (PBS-administered group). These results show that galactose is effective in weight reduction of subjects induced with obesity by high-fat diet.

### Example 4. Intraperitoneal Glucose Tolerance Test

The experimental animals were kept hungry (12-hour fasting) from 9:00 p.m. the day before the test to 9:00 a.m. the day of the test, and then a blood glucose test was performed. To keep a hungry condition, feed and straw litter were removed to induce fasting.

A 10% (w/v) glucose (in PBS) solution sterilized by 0.2µm filtration was prepared. After intraperitoneally administering 2 mg glucose/g, volume = body weight*20 (µℓ) for each individual, the mice were again put in the cage, and blood glucose levels were measured every 0, 30, 60, 90, and 120 minutes. The blood glucose tests were performed by collecting blood from the caudal vein. The intraperitoneal glucose tolerance test was performed 13 weeks after inducing obesity with a high-fat diet.

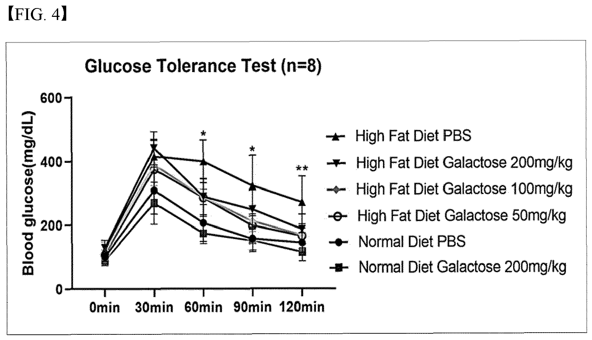

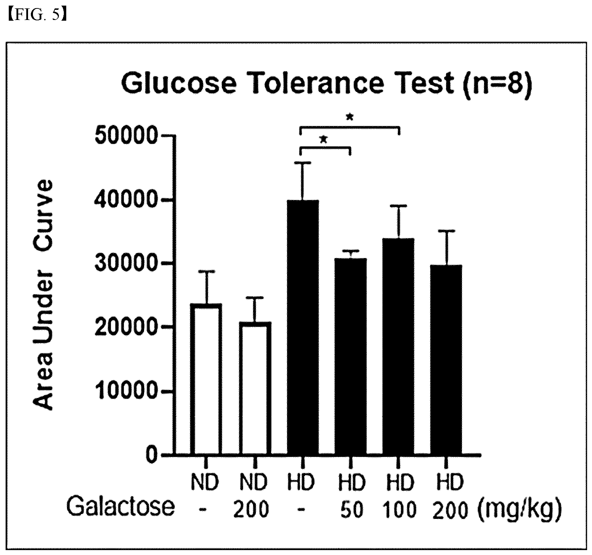

The measured blood glucose results are shown in FIG. 4, and the Area Under Curve of the graph in FIG. 4 is shown in FIG. 5. As shown in FIGS. 4 and 5, both the high-fat diet intake group (HD) and the normal diet intake group (ND) decreased in blood glucose by galactose administration, and in the high-fat diet intake group, the blood glucose level of the galactose-administered group significantly decreased as compared with a control group (PBS-administered). Particularly, when measured at 120 minutes, the galactose-administered group in the high-fat diet group showed blood sugar levels comparable to those in the normal diet group. The above results show that galactose has a blood sugar lowering effect not only in individuals induced with obesity by a high-fat diet, but also in normal individuals (normal sample intake group).

From the foregoing description, it will be apparent that various modifications and variations of the embodiment described herein can be made without departing from the scope and spirit of the present invention. In this regard, it is to be understood that the above-described embodiments are in all respects as illustrative and non-limiting. It is to be understood that the scope of the present invention is defined by the appended claims rather than the foregoing description, and that various modifications and variations made within the meaning of an equivalent of the claims of the invention and within the claims fall under the scope of the present invention.

## Claims

1. A composition for improving an intestinal microbial population, which comprises galactose as an active ingredient, and increases a proportion of at least one beneficial intestinal bacterium selected from the group consisting of *Akkermansia* bacterium, *Lactobacillus* bacterium, and *Alistipes* bacterium.

2. A pharmaceutical composition for reducing body weight, which comprises galactose as an active ingredient, and increases a proportion of at least one beneficial intestinal bacterium selected from the group consisting of *Akkermansia* bacterium, *Lactobacillus* bacterium, and *Alistipes* bacterium.

3. A pharmaceutical composition for preventing or treating obesity, which comprises galactose as an active ingredient, and increases a proportion of at least one beneficial intestinal bacterium selected from the group consisting of *Akkermansia* bacterium, *Lactobacillus* bacterium, and *Alistipes* bacterium.

4. The pharmaceutical composition according to claim 2 or 3, wherein the pharmaceutical composition is administered to a subject in which a proportion of beneficial intestinal bacterium is reduced as compared with a control group.

5. The pharmaceutical composition according to claim 2 or 3, wherein the pharmaceutical composition increases a proportion of *Akkermansia* bacterium among the beneficial intestinal bacterium, and
the pharmaceutical composition is for administering to a subject in which a proportion of *Akkermansia* bacterium among the beneficial intestinal bacterium is reduced compared to a normal weight subject.

6. The pharmaceutical composition according to claim 5, wherein the pharmaceutical composition further increases a proportion of *Alistipes* bacterium among the beneficial intestinal bacterium.

7. A food composition for reducing body weight, which comprises galactose as an active ingredient, and increases a proportion of at least one beneficial intestinal bacterium selected from the group consisting of *Akkermansia* bacterium, *Lactobacillus* bacterium, and *Alistipes* bacterium.

8. A food composition for preventing or ameliorating obesity, which comprises galactose as an active ingredient, and increases a proportion of at least one beneficial intestinal bacterium selected from the group consisting of *Akkermansia* bacterium, *Lactobacillus* bacterium, and *Alistipes* bacterium.

9. The food composition according to claim 7 or 8, wherein the food composition is administered to a subject in which a proportion of beneficial intestinal bacterium is reduced as compared with a control group.

10. The food composition according to claim 7 or 8, wherein the food composition increases a proportion of *Akkermansia* bacterium among the beneficial intestinal bacterium, and
the food composition is for administering to a subject in which a proportion of *Akkermansia* bacterium among the beneficial intestinal bacterium is reduced as compared with a normal weight subject.

11. The food composition according to claim 10, wherein the food composition further increases a proportion of *Alistipes* bacterium among the beneficial intestinal bacterium.

12. A feed composition for reducing body weight, which comprises galactose as an active ingredient, and increases a proportion of at least one beneficial intestinal bacterium selected from the group consisting of *Akkermansia* bacterium, *Lactobacillus* bacterium, and *Alistipes* bacterium.

13. A feed composition for preventing or ameliorating obesity, which comprises galactose as an active ingredient, and increases a proportion of at least one beneficial intestinal bacterium selected from the group consisting of *Akkermansia* bacterium, *Lactobacillus* bacterium, and *Alistipes* bacterium.

14. The feed composition according to claim 12 or 13, wherein the feed composition is administered to a subject in which a proportion of beneficial intestinal bacterium is reduced as compared with a control group.

15. The feed composition according to claim 12 or 13, wherein the feed composition increases a proportion of *Akkermansia* bacterium among the beneficial intestinal bacterium, and
the feed composition is for administering to a subject in which a proportion of *Akkermansia* bacterium among the beneficial intestinal bacterium is reduced as compared with a normal weight subject.

16. The feed composition according to claim 15, wherein the feed composition further increases a proportion of *Alistipes* bacterium among the beneficial intestinal bacterium.
